(19) Europäisches Patentamt
European Patent Office
Office européen des brevets

(11) Veröffentlichungsnummer: **0 180 794**
**B1**

(12) # EUROPÄISCHE PATENTSCHRIFT

(45) Veröffentlichungstag der Patentschrift: **31.05.89**

(51) Int. Cl.⁴: **C 07 D 401/06**, C 07 D 409/06, A 61 K 31/445

(21) Anmeldenummer: **85112758.9**

(22) Anmeldetag: **08.10.85**

(54) **Pyridinderivate.**

(30) Priorität: **19.10.84 DE 3438394**

(43) Veröffentlichungstag der Anmeldung: **14.05.86 Patentblatt 86/20**

(45) Bekanntmachung des Hinweises auf die Patenterteilung: **31.05.89 Patentblatt 89/22**

(84) Benannte Vertragsstaaten: **AT BE CH DE FR GB IT LI NL SE**

(56) Entgegenhaltungen:
**EP-A-0 060 176**
**EP-A-0 060 179**
**EP-A-0 101 381**
**US-A-3 125 488**
**US-A-3 546 236**

(73) Patentinhaber: **MERCK PATENT GESELLSCHAFT MIT BESCHRÄNKTER HAFTUNG, Frankfurter Strasse 250 Postfach 4119, D-6100 Darmstadt (DE)**

(72) Erfinder: **Böttcher, Henning, Dr., Soderstrasse 95, D-6100 Darmstadt (DE)**
Erfinder: **Fuchs, Andreas, Dr., Bergiusstrasse 3 B, D-3000 Hannover 51 (DE)**
Erfinder: **Seyfried, Christoph, Dr., Mathildenstrasse 6, D-6104 Seeheim- Jugenheim (DE)**

**EP 0 180 794 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft neue Pyridinderivate der Formel I

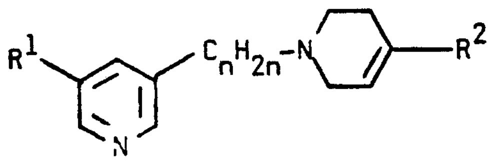

I

worin

$R^1$ und $R^2$ jeweils unsubstituierte oder ein- oder zweifach durch Alkyl, Alkoxy, F, Cl, Br, OH und/oder $CF_3$ substituierte Phenyl- oder 2- oder 3-Thienylreste und

n 1,2 oder 3

bedeuten und die Alkyl- und Alkoxygruppen jeweils 1 - 4 C-Atome besitzen,

sowie deren Salze.

Der Erfindung lag die Aufgabe zugrunde, neue Verbindungen aufzufinden, die zur Herstellung von Arzneimitteln verwendet werden können.

Es wurde gefunden, daß die genannten Substanzen bei guter Verträglichkeit wertvolle pharmakologische Eigenschaften besitzen. So zeigen sie z. B. das Zentralnervensystem beeinflussende, vorzugsweise dämpfende (z. B. sedierende, tranquillierende, neuroleptische und/oder antidepressive) Wirkungen. Im einzelnen haben die Verbindungen eine dämpfende Wirkung auf das Verhalten bei Mäusen (Methodik vgl. Irwin, Psychopharmacologia 13 (1968), 222 - 257), hemmen bei Mäusen das durch Apomorphin induzierte Kletterverhalten (Methodik vgl. Costall et al., European J. Pharmacol. 50 (1968), 39 - 50 oder induzieren contralaterales Drehverhalten in Hemiparkinson-Ratten (feststellbar nach der Methode von Ungerstedt et al., Brain Res. 24 (1970), 485 - 493), ohne daß nennenswerte kataleptische Nebenwirkungen auftreten (Methodik vgl. Dolini-Stola, Pharmakopsychiat. 6 (1973), 189 - 197). Weiterhin hemmen die Substanzen die Bindung von tritiierten Dopaminagonisten und -antagonisten an striäre Rezeptoren (feststellbar nach der Methode von Schwarcz et al., J. Neurochemistry 34 (1980), 772 - 778,und Creese et al., European J. Pharmacol. 46 (1977), 377 - 381). Zusätzlich hemmen die Verbindungen den Zungen-Kieferreflex bei der narkotisierten Ratte feststellbar in Anlehnung an die Methoden von Barnett et al., European J. Pharmacol. 21 (1973), 178 - 182, und von Ilhan et al., European J. Pharmacol. 33 (1975), 61 - 64). Weiterhin treten analgetische und blutdrucksenkende Wirkungen auf; so wird der bei katheter-tragenden wachen, spontan hypertonen Ratten (Stamm SHR/NIH-MO//CHB-EMD: Methode vgl. Weeks und Jones, Proc. Soc. Exptl. Biol. Med. 104 1960, 646 - 648) direkt gemessene arterielle Blutdruck nach intragastraler Gabe der Verbindungen gesenkt.

Verbindungen der Formel I und ihre physiologisch unbedenklichen Säureadditionssalze können daher als Arzneimittelwirkstoffe und auch als Zwischenprodukte zur Herstellung anderer Arzneimittelwirkstoffe verwendet werden.

In der US-A-3 125 488 und der EP-A-101 381 sind ähnliche Verbindungen offenbart. Für diese sind analgetische, spasmolytische und sedierende bzw. appetitzügelnde Wirkungen beschrieben, jedoch nicht die oben angegebenen Wirkungen, die für die Verbindungen der Formel I charakteristisch sind.

Gegenstand der Erfindung sind die Pyridinderivate der Formel I sowie ihre Salze.

In den Resten $R^1$ und $R^2$ bedeutet Alkyl vorzugsweise Methyl, ferner auch Ethyl, n-Propyl, Isopropyl, n-Butyl, Isobutyl, sek.-Butyl oder tert.-Butyl. Alkoxy ist vorzugsweise Methoxy, ferner auch Ethoxy, n-Propoxy, Isopropoxy, n-Butoxy, Isobutoxy, sek.-Butoxy oder tert.-Butoxy.

Die Reste $R^1$ und $R^2$ sind bevorzugt unsubstituiertes Phenyl. Falls $R^1$ bzw. $R^2$ substituierte Phenylgruppen bedeuten, so sind diese vorzugsweise einfach substituiert.

Sie können jedoch auch zweifach substituiert sein, wobei die Substituenten gleich oder verschieden sein können. Bevorzugte Substituenten an den Phenylgruppen sind Methyl, Ethyl, Methoxy, Ethoxy, F, Cl, Br und/oder OH. Im einzelnen sind $R^1$ und $R^2$ bevorzugt Phenyl, ferner o-, m- oder p-Tolyl, o-, m- oder p-Ethylphenyl, o-, m- oder p-Methoxyphenyl, o-, m- oder p-Ethoxyphenyl, o-, m- oder p-Fluorphenyl, o-, m- oder p-Chlorphenyl, o-, m- oder p-Bromphenyl, o-, m- oder p-Hydroxyphenyl, o-, m- oder p-Trifluormethylphenyl, 2,3-, 2,4-, 2,5-, 2,6-, 3,4- oder 3,5-Dimethylphenyl, 2,3-, 2,4-, 2,5-, 2,6-, 3,4- oder 3,5-Dimethoxyphenyl, 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dihydroxyphenyl, 2-Methyl-4-chlor-phenyl, 2- oder 3-Thienyl, 5-Methyl-2-thienyl.

Der Parameter n ist vorzugsweise 1, der Rest A ist vorzugsweise $-CH_2-$, weiterhin vorzugsweise $-CH(CH_3)-$, $-(CH_2)_2$ oder $-(CH_2)_3$.

Dementsprechend sind Gegenstand der Erfindung insbesondere diejenigen Verbindungen der Formel I, in denen mindestens einer der genannten Reste eine der vorstehend angegebenen, insbesondere der vorstehend angegebenen bevorzugten Bedeutungen hat. Einige bevorzugte Gruppen von Verbindungen können durch die folgenden Teilformeln Ia bis Ij ausgedrückt werden, die der Formel I entsprechen und worin die nicht näher bezeichneten Reste und Parameter die bei Formel I angegebene Bedeutung haben, worin jedoch

| | | |
|---|---|---|
| in Ia | $R^1$ | Phenyl, Tolyl, Methoxyphenyl, Fluorphenyl, Chlorphenyl, Hydroxyphenyl, Trifluormethylphenyl, Dimethoxyphenyl oder Thienyl bedeutet; |
| in Ib | $R^1$ | Phenyl, o-, m- oder p-Tolyl, m- oder p-Methoxyphenyl, p-Fluorphenyl, p-Chlorphenyl, m- oder p-Hydroxyphenyl, m-Trifluormethylphenyl, 3,4-Dimethoxyphenyl oder 2-Thienyl bedeutet; |
| in Ic | $R^1$ | Phenyl bedeutet; |
| in Id | $R^2$ | Phenyl, Tolyl, Methoxyphenyl, Fluorphenyl, Chlorphenyl, Trifluormethylphenyl, Chlor-trifluormethylphenyl oder Thienyl bedeutet; |
| in Ie | $R^2$ | Phenyl, o-, m- oder p-Tolyl, p-Methoxyphenyl, p-Fluorphenyl, p-Chlorphenyl, m-Trifluormethylphenyl oder 2- oder 3-Thienyl bedeutet; |
| in If | $R^2$ | Phenyl bedeutet; |
| in Ig | $C_nH_{2n}$ | $-CH_2-$, $-CH(CH_3)-$, $-(CH_2)_2-$ oder $(CH_2)_3-$ bedeutet; |
| in Ih | $C_nH_{2n}$ | $-CH_2-$ bedeutet; |
| in Ii | $R^1$ | Phenyl, o-, m- oder p-Tolyl, m- oder p-Methoxyphenyl, p-Fluorphenyl, p-Chlorphenyl, m- oder p-Hydroxyphenyl, m-Trifluormethylphenyl, 3,4-Dimethoxyphenyl oder 2-Thienyl, |
| | $R^2$ | Phenyl, o-, m- oder p-Tolyl, p-Methoxyphenyl, p-Fluorphenyl, p-Chlorphenyl, m-Trifluormethylphenyl oder 2- oder 3-Thienyl und |
| | $C_nH_{2n}$ | $-CH_2-$, $-CH(CH_3)-$, $-(CH_2)_2-$ oder $-(CH_2)_3-$ bedeuten; |
| in Ij | $R^1$ | Phenyl, m- oder p-Methoxyphenyl, p-Fluorphenyl, m-Hydroxyphenyl oder 3,4-Dimethoxyphenyl, |
| | $R^2$ | Phenyl und |
| | $C_nH_{2n}$ | $-CH_2-$ bedeuten. |

Einige Verbindungen der Formel I können ein oder mehrere asymmetrische Kohlenstoffatome besitzen. Sie können daher als Racemate, falls mehrere asymmetrische Kohlenstoffatome vorhanden sind, auch als Gemische mehrerer Racemate sowie in verschiedenen optisch-aktiven Formen vorliegen.

Gegenstand der Erfindung ist ferner ein Verfahren zur Herstellung der Verbindungen der Formel I sowie ihrer Salze, dadurch gekennzeichnet, daß man eine Verbindung der Formel II

$$Py\text{-}A\text{-}X^1 \qquad \text{II}$$

worin

Py den 5-$R^1$-3-pyridylrest,
A die Gruppe -$C_nH_{2n}$-,
$X^1$ X oder $NH_2$,
X Cl, Br, J, OH oder eine reaktionsfähig funktionell abgewandelte OH-Gruppe bedeuten

mit einer Verbindung der Formel III

$X^2$-$CH_2CH_2$-$CR^2$=CH-$CH_2$-$X^3$ III

worin

$X^2$ und $X^3$ gleich oder verschieden sind und, falls $X^1$ = $NH_2$ ist, jeweils X, andernfalls zusammen NH bedeuten und
$R^2$ die angegebene Bedeutung hat,

umsetzt

oder daß man eine sonst der Formel I entsprechende Verbindung, die jedoch an Stelle eines oder mehrerer Wasserstoffatome eine oder mehrere reduzierbare Gruppen und/oder eine oder mehrere zusätzliche C-C- und/oder C-N-Bindungen enthält, mit einem reduzierenden Mittel behandelt

oder daß man eine Verbindung der Formel IV

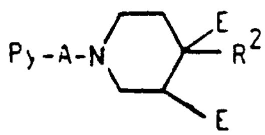

IV

worin

der eine Rest E X, CN oder $NH_2$
der andere Rest E H bedeutet und
Py, A, R und X die angegebenen Bedeutungen haben

mit einem HE-abspaltenden Mittel behandelt

und/oder daß man gegebenenfalls in einer Verbindung der Formel I eine O-Alkylgruppe unter Bildung einer DH-Gruppe spaltet

und/oder daß man eine Base der Formel I durch Behandeln mit einer Säure in eines ihrer Salze umwandelt.

Die Herstellung der Verbindungen der Formel I erfolgt im übrigen nach an sich bekannten Methoden, wie sie in der Literatur (z. B. in den Standardwerken wie Houben-Weyl, Methoden der Organischen Chemie, Georg-Thieme-Verlag, Stuttgart; Organic Reactions, John Wiley & Sons, Inc., New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu den Verbindungen der Formel I umsetzt.

In den Verbindungen der Formel II ist $X^1$ vorzugsweise X; dementsprechend sind in den Verbindungen der Formel III $X^2$ und $X^3$ vorzugsweise zusammen NH. Der Rest X ist vorzugsweise Cl oder Br; er kann jedoch auch J, OH oder eine reaktionsfähig funktionell abgewandelte OH-Gruppe bedeuten, insbesondere Alkylsulfonyloxy mit 1 - 6 (z. B. Methansulfonyloxy) oder Arylsulfonyloxy mit 8 - 10 C-Atomen (z. B. Benzolsulfonyloxy, p-Toluolsulfonyloxy, 1- oder 2-Naphthalin-sulfonyloxy).

Dementsprechend sind die Verbindungen der Formel I insbesondere durch Umsetzung von Verbindungen der Formel Py-A-Cl oder Py-A-Br mit Tetrahydropyridin-derivaten der Formel III, worin $X^2$ und $X^3$ zusammen eine NH-Gruppe bedeuten (nachstehend als IIIa bezeichnet), erhältlich.

Die Verbindungen der Formeln II und insbesondere III sind zum Teil bekannt; die nicht bekannten Verbindungen der Formeln II und III können leicht analog zu den bekannten Verbindungen hergestellt werden. Primäre Alkohole der Formel Py-A-OH sind z. B. durch Reduktion der entsprechenden Carbonsäuren oder ihrer Ester erhältlich. Behandeln mit Thionylchlorid, Bromwasserstoff, Phosphortribromid oder ähnlichen Halogenverbindungen liefert die entsprechenden Halogenide der Formel Py-A-Hal. Die entsprechenden Sulfonyloxyverbindungen sind erhältlich aus den Alkoholen Py-A-OH durch Umsetzung mit den entsprechenden Sulfonsäurechloriden. Die Jodverbindungen der Formel Py-A-J sind z. B. durch Einwirkung von Kaliumjodid auf die zugehörigen p-Toluolsulfonsäureester erhältlich. Die Amine der Formel Py-A-$NH_2$ sind z. B. aus den Halogeniden mit Phthalimidkalium oder durch Reduktion der entsprechenden Nitrile herstellbar.

Die Tetrahydropyridinderivate IIIa sind größtenteils bekannt (vgl. DE-A 2 060 816) und z. B. erhältlich durch Umsetzung von 4-Piperidon mit metallorganischen Verbindungen der Formel M-$R^2$ (worin M ein Li-Atom oder MgHal bedeutet), anschließende Hydrolyse zu den entsprechenden 4-$R^2$-4-hydroxypiperidinen sowie nachfolgende Dehydratisierung zu 4-$R^2$-3,4-dehydro-piperidinen. Verbindungen der Formel III ($X^2$ und $X^3$ = jeweils X) sind z. B. herstellbar durch Reduktion von Diestern der Formel Alkyl OOC-$CH_2$-$CR^2$=CH-COOAlkyl zu Diolen der Formel HO-$CH_2CH_2$-$CR^2$=CH-$CH_2$OH (III, $X^2$ = $X^3$ = OH) und gegebenenfalls anschließende Umsetzung mit $SOCl_2$ bzw. $PBr_3$.

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z. B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses der Aminkomponente Py-A-$NH_2$ bzw. des Piperidinderivates der Formel IIIa kann günstig sein. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°.

Es ist ferner möglich, eine Verbindung der Formel I zu erhalten, indem man ein Vorprodukt, das an Stelle von Wasserstoffatomen eine oder mehrere reduzierbare Gruppe(n) und/oder eine oder mehrere zusätzliche C-C- und/oder C-N-Bindung(en) enthält, mit einem reduzierenden Mittel behandelt, vorzugsweise bei Temperaturen zwischen -80 und +250° in Gegenwart mindestens eines inerten Lösungsmittels.

Reduzierbare (durch Wasserstoff ersetzbare) Gruppen sind insbesondere Sauerstoff in einer Carbonylgruppe, Hydroxyl, Arylsulfonyloxy (z. B. p-Toluolsulfonyloxy), N-Benzolsulfonyl, N-Benzyl oder O-Benzyl.

Es ist grundsätzlich möglich, Verbindungen, die nur eine, oder solche, die nebeneinander zwei oder mehr dieser Gruppen bzw. zusätzlichen Bindungen enthalten, reduktiv in eine Verbindung der Formel I überzuführen. Vorzugsweise bedient man sich hierzu des nascierenden Wasserstoffs oder komplexer Metallhydride, ferner der Reduktion nach Wolff-Kishner.

Bevorzugte Ausgangsstoffe für die Reduktion entsprechen der Formel V

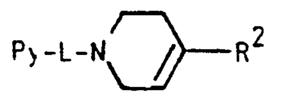

V

worin

L eine dem Rest A entsprechende Kette, worin jedoch eine oder mehrere -$CH_2$-Gruppe(n) durch -CO- und/oder ein oder mehrere Wasserstoffatome durch OH-Gruppen ersetzt sind, bedeutet.

In den Verbindungen der Formel V ist L bevorzugt -$(CH_2)_{n-1}$-CO- [im einzelnen -CO-, -$CH_2$-CO- oder -$CH_2CH_2$-CO-], ferner z. B. -$CH(CH_3)$-CO-, -$COCH_2$-, -COCO-, -$COCH_2CO$-, -CO-$CH_2CH_2$-, -$CH_2CO$-$CH_2$-, -CHOH-, -$CH_2$-CHOH-, -$(CH_2)_2$-CHOH-, -CHOH-$CH_2$-, -CHOH-CO-.

Verbindungen der Formel V sind z. B. herstellbar durch Umsetzung von IIIa mit einer Verbindung der Formel VI

Py-L-$X^1$ . VI

worin

Py, L und $X^1$ die oben angegebenen Bedeutungen haben,

unter den Bedingungen, die oben für die Umsetzung von II und III angegeben sind.

Wird als Reduktionsmittel nascierender Wasserstoff verwendet, so kann man diesen z. B. durch Behandlung von Metallen mit schwachen Säuren oder mit Basen erzeugen. So kann man z. B. ein Gemisch von Zink mit Alkalilauge oder von Eisen mit Essigsäure verwenden. Geeignet ist auch die Verwendung von Natrium oder einem anderen Alkalimetall in einem Alkohol wie Ethanol, Isopropanol, Butanol, Amyl- oder Isoamylalkohol oder Phenol. Man kann ferner eine Aluminium-Nickel-Legierung in alkalisch-wässeriger Lösung, gegebenenfalls unter Zusatz von Ethanol, verwenden. Auch Natrium- oder Aluminiumamalgam in wässerigalkoholischer oder wässeriger Lösung sind zur Erzeugung des nascierenden Wasserstoffs geeignet. Die Umsetzung kann auch in heterogener Phase durchgeführt werden, wobei man zweckmäßig eine wässerige und eine Benzol- oder Toluol-Phase verwendet.

Als Reduktionsmittel können ferner besonders vorteilhaft komplexe Metallhydride, wie $LiAlH_4$, $NaBH_4$, Diisobutyl-aluminiumhydrid oder $NaAl(OCH_2CH_2OCH_3)_2H_2$ sowie Diboran eingesetzt werden, falls erwünscht unter Zusatz von Katalysatoren wie $BF_3$, $AlCl_3$ oder LiBr. Als Lösungsmittel eignen sich hierfür insbesondere Ether wie Diethylether, Di-n-butylether, THF, Dioxan, Diglyme oder 1,2-Dimethoxyethan sowie Kohlenwasserstoffe wie Benzol. Für eine Reduktion mit $NaBH_4$ sind in erster Linie Alkohole wie Methanol oder Ethanol, ferner Wasser sowie wässerige Alkohole als Lösungsmittel geeignet. Nach diesen Methoden reduziert man vorzugsweise bei Temperaturen zwischen -80 und +150°, insbesondere zwischen etwa 0 und etwa 100°.

Besonders vorteilhaft lassen sich -CO-Gruppen in Säureamiden (z. B. solchen der Formel V, worin L -$(CH_2)_{n-1}$-CO- bedeutet) mit $LiAlH_4$ in THF bei Temperaturen zwischen etwa 0 und 66° zu $CH_2$-Gruppen reduzieren.

Es ist ferner möglich, eine oder mehrere Carbonylgruppen nach der Methode von Wolff-Kishner zu $CH_2$-Gruppen zu reduzieren, z. B. durch Behandlung mit wasserfreiem Hydrazin in absolutem Ethanol unter Druck

bei Temperaturen zwischen etwa 150 und 250°. Als Katalysator wird vorteilhaft Natriumalkoholat verwendet. Die Reduktion kann auch nach der Methode von Huang-Minlon variiert werden, indem man mit Hydrazinhydrat in einem hochsiedenden, mit Wasser mischbaren Lösungsmittel, wie Diethylenglykol oder Triethylenglykol, in Gegenwart von Alkali, wie Natriumhydroxid, umsetzt. Das Reaktionsgemisch wird in der Regel etwa 3 - 4 Stunden gekocht. Anschließend wird das Wasser abdestilliert und das gebildete Hydrazon bei Temperaturen bis zu etwa 200° zersetzt. Die Wolff-Kishner-Reduktion kann auch bei Raumtemperatur in Dimethylsulfoxid mit Hydrazin ausgeführt werden.

Man gelangt ferner zu Verbindungen der Formel I, indem man aus Verbindungen der Formel IV unter Ausbildung einer Doppelbindung HE abspaltet. Entsprechend der Definition von E kann es sich z. B. handeln um eine Abspaltung von Halogenwasserstoff, Wasser (Dehydratisierung), einer Carbonsäure oder einer anderen Säure, von Ammoniak oder von HCN. Die Ausgangsstoffe der Formel IV sind z. B. erhältlich durch Umsetzung von II ($X^1$ = X) mit einer Verbindung der Formel VII

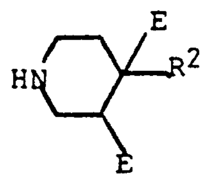

VII

worin E und $R^2$ die angegebenen Bedeutungen haben.

Falls einer der Reste E = Hal ist, kann dieser Substituent unter basischen Reaktionsbedingungen leicht eliminiert werden. Als Basen können verwendet werden: Alkalimetallhydroxide, Alkalimetallcarbonate, Alkoholate, wie z. B. Kalium-tert.-butylat, Amine, wie z. B. Dimethylanilin, Pyridin, Collidin oder Chinolin; als Lösungsmittel benutzt man z. B. Benzol,- Toluol, Cyclohexan, THF oder tert.-Butanol. Die als Basen verwendeten Amine können auch im Überschuß als Lösungsmittel eingesetzt werden. Bedeutet der eine der Reste E eine OH-Gruppe, so benutzt man als wasserabspaltende Mittel vorzugsweise Säuren wie Essigsäure, Salzsäure oder Gemische beider. Der Zusatz eines Lösungsmittels (z. B. Wasser oder Ethanol) kann von Vorteil sein. Die Eliminierung von Acyl-, Alkylsulfonyl- sowie Alkoxysulfonyl-oxy- oder Amino-Resten kann unter ähnlichen Bedingungen durchgeführt werden. Eine Eliminierung von Sulfonsäure-Resten, z. B. die der Mesylate oder Tosylate, erfolgt schonend durch Kochen in DMF oder Dimethylsulfoxid mit Alkalimetallcarbonaten, z. B. $Li_2CO_3$, oder mit Kaliumacetat. Ammoniak kann bereits durch Erhitzen der Salze der entsprechenden Aminoverbindungen (insbesondere der 4-Aminoderivate) abgespalten werden. In ähnlicher Weise kann HCN aus Verbindungen der Formel IV (eine Gruppe E = CN) durch Erhitzen abgespalten werden. Die Eliminierung von HE aus IV erfolgt allgemein bei Temperaturen zwischen 0 und etwa 250°, vorzugsweise zwischen 50 und 200°.

Weiterhin kann man gegebenenfalls eine Verbindung der Formel I nach an sich bekannten Methoden in eine andere Verbindung der Formel I umwandeln.

So können Ether (O-Alkylderivate) gespalten werden, wobei die entsprechenden Hydroxyderivate entstehen. z. B. kann man die Ether spalten durch Behandeln mit Dimethylsulfid-Bortribromid-Komplex, z. B. in Toluol, 1,2-Dichlorethan, THF oder Dimethylsulfoxid, oder durch Verschmelzen mit Pyridin- oder Anilin-hydrohalogeniden, vorzugsweise Pyridinhydrochlorid, bei etwa 150 - 250°.

Eine erhaltene Base der Formel I kann mit einer Säure in das zugehörige Säureadditionssalz übergeführt werden. Für diese Umsetzung eignen sich bevorzugt Säuren, die physiologisch unbedenkliche Salze liefern. So können anorganische Säuren verwendet werden. z. B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Säureadditionssalze, die nicht physiologisch unbedenklich sind (z. B. Pikrate), können sich zur Isolierung und Aufreinigung von Basen der Formel I eignen.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze. Diese Zubereitungen können als Arzneimittel in der Human- oder Veterinärmedizin eingesetzt werden. Als Trägersubstanzen kommen organische oder anorganische Stoffe in Frage, die sich für die enterale (z. B. orale),

parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z. B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z. B. ein oder mehrere Vitamine.

Die Verbindungen der Formel I und ihre physiologisch unbedenklichen Salze können verwendet werden bei der therapeutischen Behandlung des menschlichen oder tierischen Körpers und bei der Bekämpfung von Krankheiten, insbesondere von Schizophrenie und psychoreaktiven Störungen und Psychopathien, von Depressionen, von schweren chronischen Schmerzen und von Krankheiten, die mit erhöhtem Blutdruck einhergehen.

Weiterhin können die Verbindungen bei der Behandlung extrapyramidaler Störungen Anwendung finden.

Dabei werden die erfindungsgemäßen Substanzen in der Regel in Analogie zu bekannten, im Handel befindlichen Präparaten (Thioridazin, Dihydroergocristin) verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,003 und 10 mg/kg Körpergewicht.

Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachstehenden Beispielen bedeutet "übliche Aufarbeitung": Man gibt, falls erforderlich, Wasser hinzu, extrahiert mit Dichlormethan, trennt ab, trocknet die organische Phase über Natriumsulfat, filtriert, dampft ein und reinigt durch Chromatographie an Kieselgel und/oder durch Kristallisation. Temperaturen sind in °C angegeben.

**Beispiel 1**

Man rührt eine Lösung von 26,3 g 3-Methylsulfonyloxy-methyl-5-phenyl-pyridin (erhältlich durch Reduktion von 5-Phenylpyridin-3-carbonsäure mit $LiAlH_4$ zu 3-Hydroxymethyl-5-phenyl-pyridin und Mesylierung) und 16 g 4-Phenyl-1,2,3,6-tetrahydropyridin in 100 ml Acetonitril 12 Stunden bei 20°, arbeitet wie üblich auf und erhält 3-(4-Phenyl-1,2,3,6-tetrahydropyridyl-methyl)-5-phenyl-pyridin ("P"), F. 80 - 82°. Dihydrochlorid, F. 218 - 220°.

Analog erhält man aus den entsprechenden Methansulfonaten, Chloriden oder Bromiden:

3-(4-Phenyl-1,2,3,6-tetrahydropyridyl-methyl)-5-o-tolyl-pyridin
3-(4-Phenyl-1,2,3,6-tetrahydropyridyl-methyl)-5-m-tolyl-pyridin
3-(4-Phenyl-1,2,3,6-tetrahydropyridyl-methyl)-5-p-tolyl-pyridin
3-(4-Phenyl-1,2,3,6-tetrahydropyridyl-methyl)-5-m-methoxyphenyl-pyridin, F. 133 - 135°
3-(4-Phenyl-1,2,3,6-tetrahydropyridyl-methyl)-5-p-methoxyphenyl-pyridin, Dihydrochlorid, F. 239 - 240°
3-(4-Phenyl-1,2,3,6-tetrahydropyridyl-methyl)-5-p-fluorphenyl-pyridin, F. 128 - 129°
3-(4-Phenyl-1,2,3,6-tetrahydropyridyl-methyl)-5-p-chlorphenyl-pyridin
3-(4-Phenyl-1,2,3,6-tetrahydropyridyl-methyl)-5-m-hydroxyphenyl-pyridin, Dihydrochlorid, F. 206 - 208°
3-(4-Phenyl-1,2,3,6-tetrahydropyridyl-methyl)-5-p-hydroxyphenyl-pyridin
3-(4-Phenyl-1,2,3,6-tetrahydropyridyl-methyl)-5-m-trifluormethylphenyl-pyridin
3-(4-Phenyl-1,2,3,6-tetrahydropyridyl-methyl)-5-(3,4-dimethoxyphenyl)-pyridin Dihydrochlorid, F. 216 - 218°
3-(4-Phenyl-1,2,3,6-tetrahydropyridyl-methyl)-5-(2-thienyl)-pyridin
7-(4-o-Tolyl-1,2,3,6-tetrahydropyridyl-methyl)-5-phenyl-pyridin
3-(4-m-Tolyl-1,2,3,6-tetrahydropyridyl-methyl)-5-phenyl-pyridin
3-(4-p-Tolyl-1,2,3,6-tetrahydropyridyl-methyl)-5-phenyl-pyridin
3-(4-p-Methoxyphenyl-1,2,3,6-tetrahydropyridyl-methyl)-5-phenyl-pyridin
3-(4-p-Fluorphenyl-1,2,3,6-tetrahydropyridyl-methyl)-5-phenyl-pyridin
3-(4-p-Chlorphenyl-1,2,3,6-tetrahydropyridyl-methyl)-5-phenyl-pyridin
3-(4-m-Trifluormethylphenyl-1,2,3,6-tetrahydropyridyl-methyl)-5-phenyl-pyridin
3-[4-(4-Chlor-3-trifluormethylphenyl)-1,2,3,6-tetrahydropyridyl-methyl]-5-phenyl-pyridin
3-[4-(2-Thienyl)-1,2,3,6-tetrahydropyridyl-methyl]-5-phenyl-pyridin
3-[4-(2-Thienyl)-1,2,3,6-tetrahydropyridyl-methyl]-5-m-tolyl-pyridin
3-[4-(2-Thienyl)-1,2,3,6-tetrahydropyridyl-methyl]-5-p-tolyl-pyridin
3-[4-(2-Thienyl)-1,2,3,6-tetrahydropyridyl-methyl]-5-p-fluorphenyl-pyridin
3-[4-(3-Thienyl)-1,2,3,6-tetrahydropyridyl-methyl]-5-phenyl-pyridin

3-[1-(4-Phenyl-1,2,3,6-tetrahydropyridyl)-ethyl]-5-phenyl-pyridin
3-[1-(4-Phenyl-1,2,3,6-tetrahydropyridyl)-ethyl]-5-p-methoxyphenyl-pyridin
3-[1-(4-Phenyl-1,2,3,6-tetrahydropyridyl)-ethyl/-5-p-methoxyphenyl-pyridin
3-[2-(4-Phenyl-1,2,3,6-tetrahydropyridyl)-ethyl]-5-phenyl-pyridin
3-[2-(4-Phenyl-1,2,3,6-tetrahydropyridyl)-ethyl]-5-m-methoxyphenyl-pyridin
3-[2-(4-Phenyl-1,2,3,6-tetrahydropyridyl)-ethyl]-5-p-methoxyphenyl-pyridin
3-[3-(4-Phenyl-1,2,3,6-tetrahydropyridyl)-propyl]-5-phenyl-pyridin, Dihydrochlorid, F. 239 - 241°
3-[3-(4-Phenyl-1,2,3,6-tetrahydropyridyl)-propyl]-5-m-methoxyphenyl-pyridin
3-[3-(4-Phenyl-1,2,3,6-tetrahydropyridyl)-propyl]-5-p-methoxyphenyl-pyridin
3-(4-p-Fluorphenyl-1,2,3,6-tetrahydropyridyl-methyl)-5-p-fluorphenyl-pyridin
3-[1-(4-p-Fluorphenyl-1,2,3,6-tetrahydropyridyl)-ethyl]-5-p-fluorphenyl-pyridin
3-[2-(4-p-Fluorphenyl-1,2,3,6-tetrahydropyridyl)-ethyl]-5-p-fluorphenyl-pyridin
3-[3-(4-p-Fluorphenyl-1,2,3,6-tetrahydropyridyl)-propyl]-5-p-fluorphenyl-pyridin
3-[2-(4-(2-Thienyl)-1,2,3,6-tetrahydropyridyl)-ethyl]-5-phenyl-pyridin
3-[3-(4-(2-Thienyl)-1,2,3,6-tetrahydropyridyl)-propyl]-5-phenyl-pyridin.

**Beispiel 2**

Ein Gemisch von 1,84 g 3-Aminomethyl-5-phenylpyridin (erhältlich durch $LiAlH_4$-Reduktion von 3-Cyan-5-phenylpyridin) und 2,15 g 1,5-Dichlor-3-phenyl-2-penten in 40 ml Aceton und 40 ml Wasser wird 24 Stunden gekocht und wie üblich aufgearbeitet. Man erhält "P", F. 80 - 82°.

**Beispiel 3**

Eine Suspension von 4-Phenyl-1-(5-phenylnicotinoyl)-1,2,3,6-tetrahydropyridin (F. 81 - 83°; erhältlich durch Reaktion von 5-Phenyl-nicotinsäure mit 4-Phenyl-1,2,3,6-tetrahydropyridin in Gegenwart von Carbonyldiimidazol in THF) in 200 ml THF wird zu einer Suspension von 3,8 g $LiAlH_4$ in 200 ml THF unter Rühren hinzugetropft. Man rührt noch 2 Stunden bei 20°, arbeitet wie üblich auf und erhält "P", F. 80 - 82°.

Analog erhält man aus den entsprechenden Säureamiden die übrigen in Beispiel 1 genannten Verbindungen.

**Beispiel 4**

Man erhitzt 3,62 g 3-(4-Hydroxy-4-phenyl-1-piperidyl-methyl)-5-p-fluorphenyl-pyridin (erhältlich durch Reaktion von 3-Brommethyl-5-p-fluorphenyl-pyridin mit 4-Piperidon, anschließende Umsetzung mit $C_6H_5Li$ und Hydrolyse) mit 40 ml 1 n Salzsäure 2 Stunden auf 50°, arbeitet wie üblich auf und erhält 3-(4-Phenyl-1,2,3,6-tetrahydropyridyl-methyl)-5-p-fluorphenyl-pyridin, F. 128 - 129°.

**Beispiel 5**

Ein Gemisch von 10 g 3-(4-Phenyl-1,2,3,6-tetrahydropyridyl-methyl)-5-m-methoxyphenyl-pyridin und 10 g Pyridinhydrochlorid wird 3 Stunden bei 160° gerührt. Nach üblicher Aufarbeitung erhält man 3-(4-Phenyl-1,2,3,6-tetrahydropyridyl-methyl)-5-m-hydroxyphenyl-pyridin. Dihydrochlorid, F. 206 - 208 °.

**Beispiel 6**

Man tropft eine Suspension von 3,56 g 3-(4-Phenyl-1,2,3,6-tetrahydropyridyl-methyl)-5-m-methoxyphenyl)-pyridin in 50 ml 1,2-Dichlorethan zu einer siedenden Lösung von 15,6 g Dimethylsulfid-Bortribromid-Komplex in 50 ml 1,2-Dichlorethan, kocht noch 30 Minuten, arbeitet wie üblich auf und erhält 3-(4-Phenyl-1,2,3,6-tetrahydropyridyl-methyl)-5-m-hydroxyphenyl-pyridin, Dihydrochlorid, F. 206 - 208°.

Die nachstehenden Beispiele betreffen pharmazeutische Zubereitungen, die Amine der Formel I oder ihre Säureadditionssalze enthalten:

Beispiel A: Tabletten

Ein Gemisch von 1 kg "P"-Dihydrochlorid, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C: Kapseln

2 kg "P"-Dihydrochlorid werden in üblicher Weise in Hartgelatinekapseln gefüllt, so daß jede Kapsel 20 mg des Wirkstoffs enthält.

Beispiel D: Ampullen

Eine Lösung von 1 kg "P"-Dihydrochlorid in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

Analog sind Tabletten, Dragees, Kapseln und Ampullen erhältlich, die einen oder mehrere der übrigen Wirkstoffe der Formel I und/oder ihre physiologisch unbedenklichen Säureadditionssalze enthalten.

## Patentansprüche

1. Pyridinderivate der Formel 1.

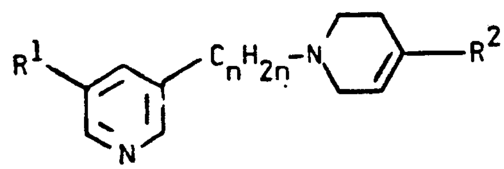

I

worin

$R^1$ und $R^2$ jeweils unsubstituierte oder ein- oder zweifach durch Alkyl, Alkoxy F, Cl, Br, OH und/oder $CF_3$ substituierte Phenyl- oder 2- oder 3-Thienylreste und

n 1, 2 oder 3

bedeuten und die Alkyl- und Alkoxygruppen jeweils 1 - 4 C-Atome besitzen,

sowie deren Salze.

2. 3-(4-Phenyl-1,2,3,6-tetrahydropyridyl-methyl)-5-phenyl-pyridin.

3. Verfahren zur Herstellung von Pyridinderivaten der Formel I sowie von deren Salzen, dadurch gekennzeichnet, daß man eine Verbindung der Formel II

$Py\text{-}A\text{-}X^1$ II

worin

| | |
|---|---|
| Py | den 5-$R^1$-3-pyridylrest, |
| A | die Gruppe $-C_nH_{2n}-$, |
| $X^1$ | X oder $NH_2$, |
| X | Cl, Br, J, OH oder eine reaktionsfähig funktionell abgewandelte OH-Gruppe bedeuten |

mit einer Verbindung der Formel III

$X^2\text{-}CH_2CH_2\text{-}CR^2=CH\text{-}CH_2\text{-}X^3$ III

worin

| | |
|---|---|
| $X^2$ und $X^3$ | gleich oder verschieden sind und, falls $X^1$ = $NH_2$ ist, jeweils X, andernfalls zusammen NH bedeuten und |
| $R^2$ | die angegebene Bedeutung hat, |

umsetzt

oder daß man eine sonst der Formel I entsprechende Verbindung, die jedoch an Stelle eines oder mehrerer Wasserstoffatome eine oder mehrere reduzierbare Gruppen und/oder eine oder mehrere zusätzliche C-C- und/oder C-N-Bindungen enthält, mit einem reduzierenden Mittel behandelt

oder daß man eine Verbindung der Formel IV

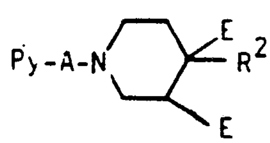

IV

worin

der eine Rest E X, CN oder $NH_2$,

der andere Rest E H bedeutet und
Py, A, R und X die angegebenen Bedeutungen haben
mit einem HE-abspaltenden Mittel behandelt

und/oder daß man gegebenenfalls in einer Verbindung der Formel I eine O-Alkylgruppe unter Bildung einer OH-Gruppe spaltet und/oder daß man eine Base der Formel I durch Behandeln mit einer Säure in eines ihrer Salze umwandelt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verbindungen der Formel I und/oder ihre physiologisch unbedenklichen Salze zur Bekämpfung von Krankheiten.

7. Verwendung von Verbindungen der Formel I und/oder ihren physiologisch unbedenklichen Salzen zur Herstellung von Arzneimitteln.

## Claims

1. Pyridine derivatives of the formula I

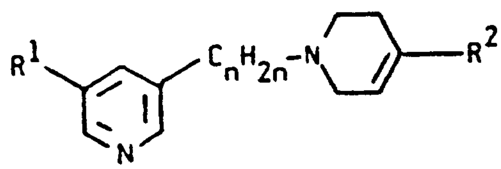

I

wherein $R^1$ and $R^2$ are each phenyl or 2- or 3-thienyl radicals which are unsubstituted or monosubstituted or disubstituted by alkyl, alkoxy, F, Cl, Br, OH and/or $CF_3$ and n is 1, 2 or 3, and the alkyl and alkoxy groups each have 1 - 4 C atoms, and salts thereof.

2. 3-(4-Phenyl-1,2,3,6-tetrahydropyridyl-methyl)-5-phenyl-pyridine.

3. Process for the preparation of pyridine derivatives of the formula I and of salts thereof, characterised in that a compound of the formula II

$$Py\text{-}A\text{-}X^1 \qquad \text{II}$$

wherein Py is the 5-$R^1$-3-pyridyl radical, A is the group $-C_nH_{2n}-$, $X^1$ is X or $NH_2$, and X is Cl, Br, I, OH or a reactively functionally modified OH group, is reacted with a compound of the formula

$$X^2\text{-}CH_2CH_2\text{-}CR^2{=}CH\text{-}CH_2\text{-}X^3 \qquad \text{III}$$

wherein $X^2$ and $X^3$ are identical or different and, if $X^1$ is $NH_2$, are each X, or otherwise together are NH, and $R^2$ has the meaning given, or in that a compound which otherwise corresponds to the formula I but contains one or more reducible groups and/or one or more additional C-C and/or C-N bonds instead of one or more hydrogen atoms, is treated with a reducing agent, or in that a compound of the formula IV

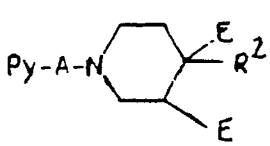

IV

wherein one radical E is X, CN or $NH_2$ and the other radical E is H and Py, A, R and X have the meanings given, is treated with an agent which splits off HE, and/or in that, if appropriate, an O-alkyl group in a compound of the formula I is split to form an OH group, and/or in that a base of the formula I is converted into one of its salts by treatment with an acid.

4. Process for the preparation of pharmaceutical formulations, characterised in that a compound of the formula I and/or one of its physiologically acceptable salts is brought into a suitable dosage form together with at least one solid, liquid or semi-liquid excipient or auxiliary and if appropriate in combination with one or more other active compound(s).

5. Pharmaceutical formulation, characterised in that it contains at least one compound of the formula I and/or one of its physiologically acceptable salts.

6. Compounds of the formula I and/or their physiologically acceptable salts for combating diseases.

7. The use of compounds of the formula I and/or their physiologically acceptable salts for the preparation of medicaments.

**Revendications**

1. Dérivés de pyridine de Formule I

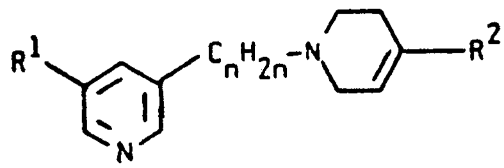

I

dans laquelle

$R^1$ et $R^2$ représentent indépendamment un reste phényle ou 2- ou 3-thiényle non substitués ou substitués une ou deux fois par un alkyle, alcoxy, F, Cl, Br, OH et/ou $CF_3$ et

n est 1, 2 ou 3

et les groupes alkyle et alcoxy possèdent chacun 1 - 4 atomes de C,

ainsi que leurs sels.

2. 3-(4-phényl-1,2,3,6-tétrahydropyridyl-méthyl)-5-phénylpyridine.

3. Procédé de préparation de dérivés de pyridine de Formule I, ainsi que de leurs sels, caractérisé en ce qu'on fait réagir un composé de Formule II

$Py\text{-}A\text{-}X^1$ II

dans laquelle

Py est le reste 5-$R^1$-3-pyridyle,
A est le groupe $-C_nH_{2n}-$,
$X^1$ est X ou $NH_2$,
X est Cl, Br, I, oH ou un groupe réactif dérivé fonctionnelle de OH

avec un composé de Formule III

$X^2\text{-}CH_2CH_2\text{-}CR^2{=}CH\text{-}CH_2\text{-}X^3$ III

dans laquelle

$X^2$ et $X^3$ sont identiques ou différents et, quand $X^1 = NH_2$, ils représentent X, sinon ils représentent ensemble NH, et
$R^2$ a la signification indiquée,

ou en ce qu'on traite avec un agent réducteur un composé correspondant à la Formule I mais qui contient à la place d'un ou plusieurs atomes d'hydrogène un ou plusieurs groupes pouvant être réduits et/ou une ou plusieurs liaisons C-C- et/ou -C-N-supplémentaires, ou en ce qu'on traite un composé de Formule IV avec un agent d'élimination de HE

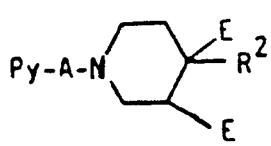

IV

dans laquelle

l'un des restes E est X, CN ou $NH_2$,
l'autre reste E est H et
Py, A, R et X ont les significations données, et/ou en ce que le cas échéant, dans un composé de Formule I, on élimine un groupe O-alkyle en formant un groupe OH,

et/ou en ce qu'on transforme une base de Formule I par traitement avec un acide en un de ses sels.

4. Procédé de fabrication de préparations pharmaceutiques, caractérisé en ce qu'on place sous une forme de dosage appropriée un composé de Formule I et/ou un de ses sels physiologiquement acceptable avec au moins un véhicule ou produit auxiliaire solide, liquide ou semi-liquide en combinaison avec un ou plusieurs autres principes actifs.

5. Préparation pharmaceutique, caractérisée en ce qu'elle contient au moins un composé de Formule I et/ou

un de ses sels physiologiquement acceptables.

6. Composés de Formule I et/ou leurs sels physiologiquement acceptables pour combattre des maladies.

7. Utilisation de composés de Formule I et/ou de leurs sels physiologiquement acceptables pour préparer de médicaments.